# NEW EUROPEAN PATENT SPECIFICATION

(11) **EP 0 165 301 B2**
(45) Date of publication and mention of the opposition decision: **14.12.1994**
(45) Mention of the grant of the patent: 03.04.1991
(21) Application number: 85900380.8
(22) Date of filing: 07.12.1984
(51) Int. Cl.: A61B 17/36

(54) **EXCIMER LASER FOR MEDICAL TREATMENT ON ORGANIC TISSUE IN BIOLOCICAL SYSTEMS AT A PATHOLOGICAL SITUS**
LASER ZUR MEDIZINISCHEN BEHANDLUNG KRANKER BEREICHE ORGANISCHEN GEWEBES
LASER EXCISEUR POUR TRAITEMENT MEDICAL SUR UN TISSU ORGANIQUE DANS DES SYSTEMES BIOLOGIQUES A UN SITE PATHOLOGIQUE

(30) Priority: 08.12.1983 US 559430
(43) Date of publication of application: 27.12.1985
(73) Proprietor: Cedars-Sinai Medical Center, Los Angeles, California 90048-0750 (US)
(72) Inventor: GRUNDFEST, Warren, Scott, Los Angeles, CA 90024 (US)
(74) Representative: Lehn, Werner, Dipl.-Ing.
(86) International application number: US8402000
(87) International publication number: WO8502532

(56) References cited:
- EP-A- 0 111 060
- DE-A- 3 046 894
- GB-A- 964 567
- US-A- 3 769 963
- US-A- 3 821 510
- US-A- 3 858 577
- US-A- 4 146 019
- US-A- 4 207 874
- US-A- 4 334 199
- US-A- 4 393 505
- Health Physics, Vol. 40, May 1981, TABOADA et al, "Response Of The Corneal Epithelium To KrF Excimer Laser Pulses", see pages 677-683.
- New York Times, September 1982, "Laser Seen As Hope In Avoiding Surgery For Blocked Artery".
- American Journal Of Cardiology, Vol. 50, December 1982, CHOY et al, "Laser Coronary Angioplasty: "Experience With 9 Cadaver Hearts", see pages 1209-1211.
- American Journal Of Cardiology, Vol. 50, December 1982, CHOY et al, "Transluminal Laser Catheter Angioplasty", see pages 1206-1208.
- Ieee Press, June 1973, DISLICH et al, "Light Guide System For The Ultraviolet Region Of The Spectrum", see pages 94-99.
- American Journal of Ophthalmology, December 1983, TROKEL et al, "Excimer Laser Surgery Of The Cornea", see pages 710-715.
- THE LASER GUIDE BOOK, (J. HECHT), Mc GRAW-HILL BOOK CP4, 1986, pages 176-177.
- Scientific American, Vol. 219, No. 3, September 1968, SCHAWLOW, "Laser Light", see pages 120-134.
- Scientific American, Vol. 240, No. 5, May 1979, "Laser Chemistry", see pages 114-128.
- LASER FOCUS, vol. 19, No. 5, May 1983, pages 62, 64, 66, R. SRINIVASAN et al.: FAR-UV photoetching or organic material.

## Description

Having found a host of uses in industry, surveying and communications, lasers at present have found only limited utility in the medical arena. The major problem encountered in medical apparatus using currently available Argon lon, Neodynium Yttrium-Aluminium-Garnet (Nd-YAG) and carbon dioxide lasers is uncontrolled damage to normal healthy tissue adjacent to the pathological area at which the laser is directed. The effect of these lasers on human tissues can best be described as primarily a thermal process. As the laser irradiates tissue, it causes the tissue temperature to increase. The tissue directly beneath the incident beam blanches and then melts. A small crater appears at the radiation situs. The liquid produced as a result of the tissue melting boils, producing explosive bubbles. As the molten tissue continues to vapourize, the crater depends with the crater margins progressively expanding. The heat generated at the radiation situs is conducted to surrounding areas of tissue thereby producing thermal injury to tissue areas remote from the radiation situs. (See Abela et al, Am J Cardiology Vol 50, p 1199 (1982)). Moreover, the extent of histologic injury adjacent to the crater varies direcly with the energy delivered. (See Gerrity et al, Cardiovasc Surg, Vol 85, p 409 (1983) and Castro et al Annals of Plastic Surgery, Vol 9, No 33, p 221 (1982)). Furthermore, the longer the area is exposed to the radiation, the greater the resulting adjacent tissue damage will be. The locally generated thermal damage precludes medical applications of lasers in procedures in which precise, cell-by-cell tissue ablation is contemplated.

In 1981, the first reported study undertaken to determine the effect of laser irradiation focused on human cadaver coronary arteries was performed. (See Lee, et al, AM. Heart J., Vol. 102, No. 6, p. 1074 (1981)). This study was designed to test the potential effects of laser irradiation on the removal of atherosclerotic plaques in coronary arteries of humans.

This and subsequent studies suffered from the same complications referred to above, namely, carbonization or charring of concentric areas of surrounding tissue in proportion to the energy delivered and coagulation necrosis. The energy required to abate tissue and achieve desired effects, namely recanalization of the vessel, also leads in some cases to perforation of the vessel wall.

As a result of these complications, clinical utilization of such lasers has largely been limited to photocoagulation and destruction of tumors and skin lesions.

Photocoagulation is the process of using laser energy to coagulate and/or denature proteins and is used in ophthalmology as a treatment for ocular disorders such as detached retinas. The basic reaction in photocoagulation involves the conversion of radiant energy of the laser into thermal energy in the tissue thereby producing a localized lesion of photocoagulated or denatured protein substances. The resulting scar provides a base upon which the detached retina adheres. Photocoagulation is also utilized in the treatment of various vascular abnormalities and to cauterize broken blood vessels. The blue-green light emitted by the argon laser is particularly effective in this procedure.

Lasers are also effective in the destruction of tumors and skin lesions. In this capacity, the laser operates to vaporize the pathological tissue layer by layer. As such, its thermal destructive effects produce the beneficial effect of destroying the tumor mass. However, even where the tumor mass is highly localized, heat from the lased tumor tissue is conducted to adjacent healthy tissue presenting the same problems mentioned above. In all these limited medical applications, the respective procedures rely on the laser's thermal effects. But it is precisely those thermal effects which foreclose successful use of Argon, Nd-YAG and carbon dioxide lasers in a host of other procedures.

For example, thermal destruction of even minute areas of adjacent brain, spinal, nevous system, coronary and other delicate tissues would produce devastating effects thereby effectively reducing the clinical utility of such lasers in procedures involving these tissues. For surgical procedures involving these types of tissues, precise cell-by-cell tissue ablation without the thermal destructive effects is highly desirable.

In Laser Focus, Vol 19, No 5, May 1983 pages 62 to 66 in an article by R Srinivasen et al entitled "For UV-photoetching of organic material" there is described a pulsed organic fluoride excimer laser which generates intense beams of 193 nm radiation to etch the surface of biological material eg human hair in a controlled manner. The implication of the tests carried out is that wavelengths in excess of 200 nm would be likely to damage external tissue.

"GB-A-0964567 describes an apparatus in accordance with the preamble of claim 1.

An object of the present invention is to provide an improved laser apparatus and delivery system for use in biological systems.

According to the present invention there is provided a laser apparatus comprising a laser, control means for controlling the operation of the laser, optical means coupled to the output of the laser and delivery means for delivering the laser radiation to a desired site, the delivery means comprising a medical instrument for penetrating the human body, and the optical means passing through said medical instrument characterized in that the laser is an excimer laser; and the control means controls the excimer laser to generate a pulsed output at a wavelength of 308 nm, to carry out ablative photodecomposition in an internal part of the human body.

The laser apparatus to be hereinafter described emits radiation in the near ultra violet wavelength range and provides for its delivery to biological tissues without the inherent complications and thermal tissue injury which inhibit fuller utilization of conventional Argon, Nd-YAG and C02 lasers in biological systems.

While the precise process by which this laser apparatus operates in biological systems has not been fully elucidated, two postulated mechanisms have been proposed. In the first postulate known as photochemical desorption, the excimer laser excites the molecules in biological tissues to energy levels which exceed the ionization potential of many organic molecules causing disruption of the chemical bonds which hold these molecules together. (See Srinivasan, et al, J.A.C.S., Vol. 103, p. 5245 (1981)). As the chemical bonds disrupt, the molecular fragments are ejected carrying away the excess energy of the photon pulse thereby preventing a temperature rise in the tissues surrounding the area of the incident beam.

In the second postulate, the excimer laser as a result of its short pulse duration capability, has the capacity to vaporize the incident tissue at such a rapid rate that the rate of tissue destruction exceeds the ability of that tissue to conduct the heat away to surrounding tissue. The heating is so rapid that it is only superficial, and the vaporization carries away the heat before it has time to penetrate below the surface by conduction.

Both postulated mechanisms lead to the same conclusion. Since the temperature in the surrounding tissues does not increase appreciably, the damage to the cells in the area adjacent to the incident beam is minimal. The tissue damage (including carbonization or charring, coagulation necrosis and vacuolization secondary to blast or sonic injury) which is characteristic of the Argon, Nd-Yag and C02 lasers used in biological systems is drastically reduced. The result is a clean, almost scalpel-like incision in the area of the laser beam.

The clinical implications of a laser apparatus with the described capabilities are far-reaching. The methods of treatment which the invention contemplates may obviate the need for the traumatic and highly invasive procedures utilized in, for example, the treatment of coronary occlusions, biliary and urological obstructions, and in orthopedics, the drilling of bone.

In one embodiment a fiberoptic waveguide which is designed to convey laser radiation of specified wavelength is delivered to a variety of pathological sites in the body without the need for elaborate and more invasive surgical procedures. This can be accomplished by the insertion of a hollow catheter means into the vasculature of the patient's arm. When inserted, the catheter means follows the contours of the blood vessel into which it is inserted until it arrives at the area of the pathological situs, for example, the coronary arteries. The fiberoptic waveguide is then inserted into the hollow catheter means for ultimate delivery to the area of occlusion in the coronary arteries. The fiberoptic is secured about the mouth of the catheter body by means of a coupler. Laser radiation of specified wavelength (308 nanometers is then delivered from the excitation chamber through a fused silica fiberoptic waveguide for ultimate delivery to the atherosclerotic plaque which adheres to the intimal wall of the coronary arteries and is composed of cholesterol, esterified fatty acids and calcium salts with a small amount of protein. When the energy is delivered to the pathological site the plaque is vaporized with minimal damage to surrounding tissues.

The excimer laser does not operate by the same thermal processes which critically inhibit the use of conventional lasers on organic tissue. During excimer irradiation of organic tissue, small wedge- shaped craters are formed without bubbling of tissue, smoke, carbonization or charring of the tissue margins and no late lateral expansion of the zone of tissue injury. Histologic examination reveals that the depth of penetration of the incident beam through the tissues varies directly with the number of pulses. Significantly, the width of the crater as measured at its widest point does not increase, but remains relatively constant, independent of the number of pulses. The relative lack of thermal destructive tissue effects and the clean, scalpel-like incision produced by the excimer laser allows for a degree of precision in the surgical capability of lasers which is unprecedented. In comparison to the uncontrolled tissue destruction caused by vaporization secondary to thermal dessication by lasers such as the Argon laser, exquisite control of tissue destruction is obtained using the excimer laser.

In another embodiment the fiberoptic waveguide is delivered to the pathological situs by endoscopic means. The endoscopic means provides access to the surgical situs which would otherwise be accessible only by general surgical techniques. The fact that the subject procedure does not require surgical incision and exposure of the pathological situs obviates the need for general anesthesia, and the procedure may be performed by using a local anesthetic. For example, in one embodiment, damaged cartilage in a patient's knee is made expediently accessible by insertion of the fused silica fiberoptic waveguide through an arthroscope. The damaged cartilage can be visualized through fiberoptic bundles inserted along with the fiberoptic waveguide for conveyance of the laser radiation. When the target site is visualized, the laser radiation is emitted through the fiberoptic and the torn or damaged tissue is vaporized.

In orthopedic applications the very precise cutting capabilities of the excimer laser alleviates the need for conventional drilling procedures in the cutting of bone. The invention is also useful in breaking up renal calculi or kidney stones. Also, the precise, cell-by-cell tissue ablation which the invention contemplates allows for the use of the invention in the delicate tissues of the brain and nervous system.

An excimer laser apparatus embodying the present invention will now be described, by way of example, with reference to the accompanying diagrammatic drawings, in which:
Fig 1 is a partially exploded perspective view of a excimer laser apparatus with a fused silica fiberoptic waveguide and catheter delivery system according to one embodiment of the present invention;
Fig 2 is a perspective view of an excimer laser apparatus and fiberoptic endoscopic delivery system according to one embodiment of the present invention; and
Fig 3 is a perspective view of an excimer laser apparatus and focusing lens delivery system according to one embodiment of the present invention.

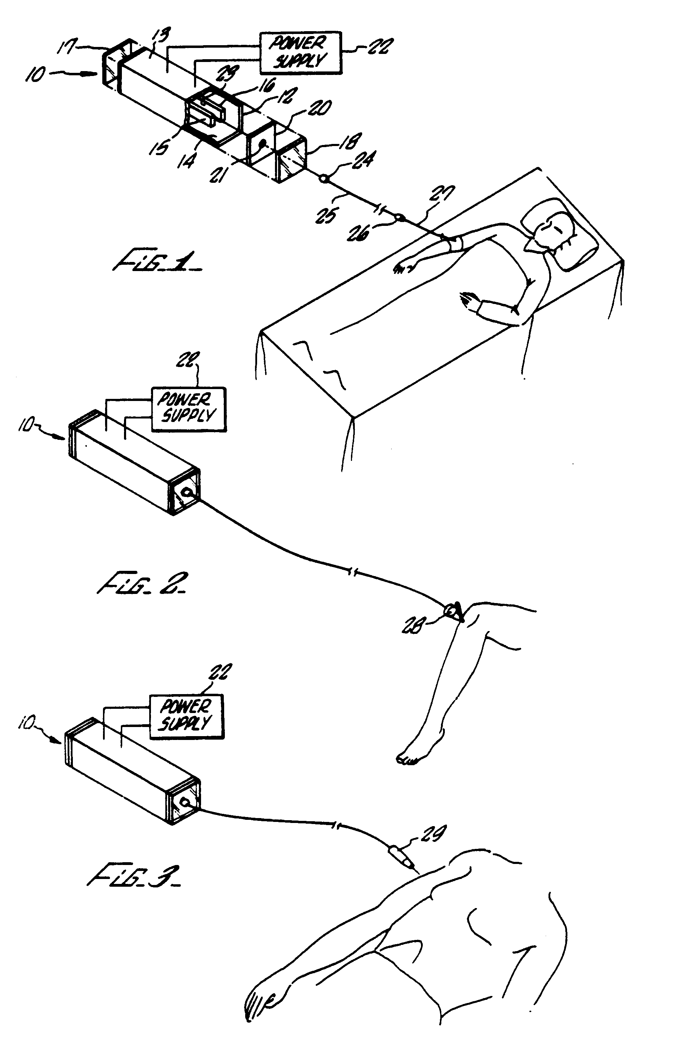

while the invention has application with utility and advantage to a multiplicity of clinical procedures, the following will describe the invention in one such application. Referring now to Fig 1, there is shown an excimer laser apparatus 10 having a power source 22, a sealed metallic housing with top 13, bottom 14 and side plates 11 and 12 composed of non-conductive metal and end walls 19 and 20 defining therein a sealed, gas-tight laser excitation chamber 23 for dipersion therein of a lasing gas mixture. The end walls disclose an aperture 21 which is aligned with the electrical discharge zone as defined by the area between a pair of parallel electrodes 15 and 16 located inside the laser housing. The aperture 21 is disposed for transmission therethrough of laser radiation from the internal excitation chamber 23. The excitation chamber 23 contains a lasing gas medium which upon excitation produces an excimer (excited dimer). The lasing gas medium is composed of the noble gas Xe and the halogen CI

At opposite ends of the laser body are mirrors 17 and 18 disposed for alignment with window 21 and electrical discharge zone. Mirror 17 is totally reflective for complete reflection of laser radiation whereas mirror 18 is only partially reflective allowing for transmission of laser output energy through the mirrored surface for ultimate delivery to the pathological site. The excited laser gas medium emits radiation through the aperture 21 and the partially transmissive mirror 18 into a fused silica fiberoptic waveguide 25 which is operatively attached 24 to the laser body in alignment with the laser discharge zone. The radiation is then conveyed via the fiberoptic for delivery to the pathological situs.

The fiberoptic may be delivered by means of a hollow catheter 27 disposed for insertion therein of the fiberoptic waveguide 25. The fiberoptic 25 is secured about the mouth of said hollow catheter by means of a coupler 26. The hollow catheter 27 extends from a point outside the body for reception therein of the fiberoptic 25 and extends to the area of the pathological site for sealed metallic housing with top 13, bottom 14 and side plates 11 and 12 composed of non-conductive metal and end walls 19 and 20 defining therein a sealed, gas-tight laser excitation chamber 23 for dispersion therein of a invention, the catheter 27 may be delivered by means of insertion through the vasculature in the patient's arm and directed to the coronary arteries for radiation and vaporization of atherosclerotic plaque which adheres to the intimal surface of the arteries.

FIG. 2 shows another embodiment of the invention whereby the fiberoptic waveguide 25 conveys the laser radiation from the optical discharge zone to the pathological site by endoscopic means 28. The endoscopic means 28 may consist of an arthroscope disposed for insertion therein of said fiberoptic waveguide 25 for ultimate delivery to, for example, damaged cartilage in a patient's knee.

Fig. 3 depicts the delivery of laser radiation through a focusing lens 29 for ultimate delivery to any external pathological site or to a surgically exposed pathological site.

The laser includes control means which controls the wavelength of the laser to be 308nm. Also the control means acts to pulse the output of the laser.

From the above description it will be understood that there has been provided improved laser apparatus for delivery of radiation to biological systems and improved methods of medical treatment using laser radiation. The advantages of the invention are obtained from the use of excimer laser apparatus on biological tissue with a compatible delivery system for ultimate conveyance of the laser radiation to the pathological site in the biological system.

## Claims

1. A laser apparatus comprising a laser (10), control means for controlling the operation of the laser, optical means (25) coupled to the output of the laser (10) and delivery means for delivering the laser radiation to a desired site, the delivery means comprising a medical instrument (27 or 28) for penetrating the human body, and the optical means (25) passing through said medical instrument (27 or 28) characterized in that the laser is an excimer laser; and the control means controls the excimer laser to generate a pulsed output at a wavelength of 308 nm, to carry out ablative photodecomposition in an internal part of the human body.

2. Apparatus according to Claim 1 characterised in that said medical instrument comprises an endoscope (28) designated to be able to direct laser energy from the optical means (25) to incise human tissue.

3. Apparatus according to Claim 1 characterised in that said medical instrument is a catheter (27) designated to be able to irradiate with laser energy from the optical means (25) atherosclerotic obstructions in blood vessels.

4. Apparatus according to any preceding claim characterised in that said optical means (25) includes a fused silica fibreoptic waveguide.

5. Apparatus according to Claim 4 characterised in that said optical means (25) includes a focusing lens (29).

6. Apparatus according to any preceding claim characterised in that the excimer laser (10) includes Xenon as a lasing gas.

7. Apparatus according to any preceding claim characterised in that said excimer laser (to) includes chlorene as a lasing gas.

## Patentansprüche

1. Laservorrichtung mit einem Laser (10), einer Steuereinrichtung zum Steuern des Laserbetriebs, einer an den Ausgang des Lasers (10) angeschlossenen optischen Einrichtung (25) und einer Zuführeinrichtung zum Zuführen des Laserstrahls an eine gewünschte Stelle, wobei die Zuführeinrichtung ein medizinisches Instrument (27 oder 28) zum Eindringen in den menschlichen Körper aufweist und die optische Einrichtung (25) durch das medizinische Instrument (27 oder 28) hindurchgeführt ist, dadurch gekennzeichnet, daß der Laser ein Exzimerlaser ist; und die Steuereinrichtung den Exzimerlaser derart steuert, daß dieser einen Ausgangsimpuls mit einer Wellenlänge von 308 nm erzeugt, um eine ablative fotochemische Zerlegung in einem inneren Teil des menschlichen Körpers auszuführen.

2. Vorrichtung nach Anspruch 1, dadurch gekennzeichnet, daß das medizinische Instrument ein Endoskop (28) aufweist, welches derart ausgebildet ist, daß die Laserenergie von der optischen Einrichtung (25) so auf menschliches Gewebe richtbar ist, daß dieses geschnitten wird.

3. Vorrichtung nach Anspruch 1, dadurch gekennzeichnet, daß das medizinische Instrument ein Katheter (27) ist, welcher darart ausgebildet ist, daß mit der Laserenergie von der optischen Einrichtung (25) arteriosklerotische Verstopfungen in Blutgefäßen bestrahlbar sind.

4. Vorrichtung nach einem der vorhergehenden Ansprüche, dadurch gekennzeichnet, daß die optische Einrichtung (25) einen Lichtleiter aus geschmlozenem Silika aufweist.

5. Vorrichtung nach Anspruch 4, dadurch gekennzeichnet, daß die optische Einrichtung (25) eine Fokussierlinse (29) aufweist.

6. Vorrichtung nach einem der vorhergehenden Ansprüche, dadurch gekennzeichnet, daß der Exzimerlaser (10) Xenon als Lasergas aufweist.

7. Vorrichtung nach einem der vorhergehenden Ansprüche, dadurch gekennzeichnet, daß der Exzimerlaser (10) Chlor als Lasergas aufweist.

## Revendications

1. Appareil à laser comprenant un laser (10), un moyen de commande pour la commande du fonctionnement du laser, un moyen optique (25) couplé avec la sortie du laser (10) et un moyen de conduite pour conduire le rayon laser jusqu'à un site désiré, le moyen de conduite comprenant un instrument médical (27 ou 28) destiné à pénétrer dans le corps humain et le moyen optique (25) passant à travers ledit instrument médical (27 ou 28), caractérisé en ce que le laser est un laser à excimère et le moyen de commande commande le laser à excimère pour la génération d'un signal de sortie pulsé à une longueur d'onde de 308 nm, pour réaliser une photodécomposi- tion ablative sur une partie interne du corps humain.

2. Appareil selon la revendication 1, caractérisé en ce que l'instrument médical comprend un endoscope (28) conçu pour être capable de diriger l'énergie du laser à partir du moyen optique (25) pour l'incision de tissus humains.

3. Appareil selon la revendication 1, caractérisé en ce que l'instrument médical est un cathéter (27) conçu pour être capable d'irradier sur des obstructions athérosclérotiques dans des vaisseaux sanguins de l'énergie du laser provenant du moyen optique (25).

4. Appareil selon l'une quelconque des revendications précédentes, caractérisé en ce que le moyen optique (25) comprend un guide d'onde à fibre optique en silice fondue.

5. Appareil selon la revendication 4, caractérisé en ce que le moyen optique (25) comprend une lentille convergente (29).

6. Appareil selon l'une quelconque des revendications précédentes caractérisé en ce que le laser à excimère (10) contient du xénon comme gaz d'émission du rayon laser.

7. Appareil selon l'une quelconque des revendications précédentes, caractérisé en ce que le laser à excimère (10) contient du chlorène comme gaz d'émission du rayon laser.
